# EUROPEAN PATENT APPLICATION

(11) **EP 4 039 175 A1**
(43) Date of publication of application: **10.08.2022**
(21) Application number: 21305145.1
(22) Date of filing: 04.02.2021
(51) Int. Cl.: A61B 5/00, A61M 37/00

(54) **ON-SKIN CIRCUIT ASSISTANCE**

(71) Applicant: Société BIC, 92110 Clichy (FR)
(72) Inventor: DUFFY, David, 8003 Zürich (CH); ELLIOTT-BOWMAN, Bernadette, Leatherhead, Surrey, KT22 8LR (GB)
(74) Representative: Peterreins Schley

(57) **Abstract**

A computer-implemented method for designing a user-specific electronic tattoo that comprises obtaining a user-input is provided. The user-input includes one or more desired functions of the electronic tattoo and a desired appearance of the electronic tattoo. The method further comprises generating a design of the electronic tattoo based on the user-input. The generation of the design of the electronic tattoo includes determining an electronic circuit design which is capable of achieving the one or more desired functions. The generation further includes designing a final image of the electronic tattoo that mimics the desired appearance and incorporates the electronic circuit design.

## Description

### Technical Field

The present disclosure relates to a computer-implemented method for designing a user-specific electronic tattoo and to a tattoo design system configured to perform the method.

### Background

The field of wearable electronics is growing. Another approach of providing a user with electronic functions are electronic tattoos. Electronic tattoos regularly comprise an electronic circuit consisting of circuit components and conductive ink such as graphite. Various functions may be integrated into electronic tattoos, such as remote control, medical functions or displaying information. Electronic tattoos may be applied on a skin of a user (on-skin) or any other surface of, e.g. a furniture surface, building surface or animal surface.

The electronic circuit of the electronic tattoo, also referred to as on-skin electronics or on-skin biomedical device, having advanced sensor functions can be printed at home using only electronic ink or even pencil and paper. These easy-to-produce sensors can be used to gather, inter alia, surface electromyography signals (sEMG) for, e.g., gesture detection, Electrodermal Activity signals (EDA) for, e.g., emotion sensing and Electrocardiography signals (ECG) for, e.g., athletic performance. More complex components such as displays can be pre-manufactured using flexible biocompatible materials.

The object of the present disclosure is to provide a method and system which enables a user to create an electronic tattoo based on user preferences.

### Summary

The present disclosure relates to a computer-implemented method as defined in claim 1, a storage media as defined in claim 14 and a tattoo design system as defined in claim 15. The dependent configurations depict further embodiments of the present disclosure.

The computer-implemented method for designing a user-specific electronic tattoo comprises obtaining a user-input. The user-input includes one or more desired functions of the electronic tattoo and a desired appearance of the electronic tattoo. The method further comprises generating a design of the electronic tattoo based on the user-input. The generation of the design of the electronic tattoo includes determining an electronic circuit design which is capable of achieving the one or more desired functions. The generation of the design of the electronic tattoo further includes designing a final image of the electronic tattoo that is configured to mimic the desired appearance and incorporates the electronic circuit design. The presently disclosed method can enable a user, particularly an unskilled user, to produce electronic tattoos that are both visually appealing and functional. Designing a final image of the electronic tattoo can be understood as creating an electronic tattoo design which then may be produced by a user. The desired functions may include non-medical and medical applications.

In aspects, the user-input may be provided by a user via a user-interface. In examples, the final image of the electronic tattoo may be provided, e.g. visualized, via the user-interface. In examples, user instructions for creation of the electronic tattoo may be provided, e.g. visualized, via the user-interface. In examples, audio instructions may be given to the user.

In aspects, the one or more desired functions may be selected from a pre-set list of possible functions. In examples, the one or more desired functions may be created by a user. The desired functions may, for instance, include one or more physiological sensors, one or more visual displays, one or more haptic or other feedback system, or any other function known in the art.

In aspects, the desired appearance may be provided by uploading an image file. Additionally or alternatively, the desired appearance may be provided by selecting an image file from a tattoo image database which stores existing tattoo designs. Existing tattoo designs may comprise pre-defined examples (e.g. default examples) or uploaded user-created designs. Additionally or alternatively, salient features, e.g. visual and/or aesthetic features, of the image file may be identified and extracted by image recognition. In examples, the visual and/or aesthetic features may be conveyed into a target image.

Additionally or alternatively to the previous aspect, the desired appearance may be provided by uploading basic preferences, particularly user preferences. The preferences may regard one or more of shape, size, colouration, style, visual features and/or aesthetic features. In examples, the preferences, particularly the visual and/or aesthetic features, may be conveyed into a target image.

In aspects, the user-input on the desired appearance may include constraints on the desired appearance. The constraints may define a degree of how close the desired appearance is to be met. For instance, constraint options may include closest possible match or iterative match.

In aspects, determining the electronic circuit design may comprise applying a circuit design algorithm. The circuit design algorithm may be configured to generate circuit parameters which are necessary for achieving the one or more desired functions. Generating circuit parameters may include identifying circuit component candidates which fulfill the desired functions. Furthermore, generating circuit parameters may include identifying electrical connections between circuit component candidates. In examples, constraints such as circuit layout, circuit component separation, electrical connection length and/or required circuit component location may be identified.

In aspects, identifying circuit component candidates may include determining circuit component candidates from circuit components which are stored in a component database and which fulfill the desired functions. In examples, optimum circuit component candidates may be pre-selected from the circuit component candidates based on predefined selection metrics. In examples, the predefined selection metrics for selecting the optimum circuit components may include one or more of dimensional fit to the desired or determined location, efficiency and/or performance of the electronic circuit, availability of circuit components, and price of circuit components. Selecting the optimum circuit components may be prioritized by one or more of the predefined selection metrics. Particularly, selecting the optimum circuit components may be prioritized by an order of two or more of predefined selection metrics.

In aspects, determining circuit component candidates may include obtaining information from the component database regarding one or more of power requirements, resistance, voltage, current properties, circuit component connection type, and/or circuit component size.

In aspects, designing the final image of the electronic tattoo may comprise applying a visual design algorithm. The visual design algorithm may be configured to obtain the previously generated circuit parameters. In examples, a target image which includes determining visual and/or aesthetic features of the desired appearance may be created. In examples, circuit parameters may be incorporated into the target image. In examples, the final image of the electronic tattoo may be created by filling out part of or all remaining sections of the target image with non-conductive tattoo component ink.

In aspects, incorporating the circuit parameters into the target image may include selecting final circuit components. In examples, incorporating the circuit parameters into the target image may include placing the final circuit components into the target image. In examples, the final circuit components may be defined as electronic tattoo component devices. In examples, the identified electrical connections may be placed into the target image. In examples, the identified electrical connections may be defined as conductive tattoo component ink.

In aspects, selecting final circuit components may include comparing visual and/or aesthetic features of the circuit component candidates with the visual and/or aesthetic features of the desired appearance. The visual and/or aesthetic features of the circuit component candidates may include one or more of colour, shape and/or size of the circuit component candidates. The visual and/or aesthetic features of the circuit component candidates may be obtained from the component database based on the circuit parameters. Determining the visual and/or aesthetic features of the desired appearance may include applying machine vision techniques analyzing aesthetic/artistic style, colour palette and/or image features of an image file provided by the user as user-input on the desired appearance. Alternatively or additionally, determining the visual and/or aesthetic features of the desired appearance may include applying semantics analysis of a non-image based user-input, such as textual input, on the desired appearance. Selecting final circuit components may further include selecting those circuit components from the circuit component candidates which closest match the visual and/or aesthetic features of the desired appearance based on the comparison. Alternatively or additionally, sections of the target image may be altered to match the visual and/or aesthetic features of those circuit components for which no circuit component candidates are present which match the visual and/or aesthetic features of the desired appearance.

In aspects, a user can adjust or edit the aesthetic and/or visual features of the desired appearance at any stage during the method. Particularly, the aesthetic and/or visual features of the target image or final image may be adjusted by a user. In some aspects, the user may adjust or edit the desired functions of the electronic tattoo at any stage during the method.

In aspects, the computer-implemented method may further comprise generating user instructions for producing the electronic tattoo based on the final image of the electronic tattoo. The user instructions may be communicated to the user. In examples, the user may be provided with a list of electronic tattoo components required to produce the electronic tattoo. The electronic tattoo components may comprise one or more of circuit components, conductive inks, non-conductive inks and a base layer. In examples, the electronic tattoo components may comprise at least circuit components and conductive inks.

In aspects, generating user instructions may include applying an instructions generation algorithm. The instructions generation algorithm may be configured to generate a text-based list of instructions in natural language. In examples, text generation machine learning techniques may be user to generate the text-based list. Additionally or alternatively to the text-based list, a sequence of step-by-step images of the electronic tattoo may be generated. Additionally or alternatively, the final image of the electronic tattoo may be projected at a desired location. In some aspects, the projection may include applying augmented reality technologies overlay or similar technologies.

In aspects, the user instructions may be communicated to the user acoustically. Alternatively or additionally the user instructions may be communicated to the user visually. In some aspects, communicating the user instructions may include projecting an image of the electronic tattoo at a desired location. In some aspects, the projection may include applying augmented reality technologies overlay or similar technologies.

In aspects, the computer-implemented method may further comprise obtaining a target location for application of the electronic tattoo. In examples, the method may comprise collecting physiological data on the target location. In examples, the collected physiological data may be considered during determining the electronic circuit design. Additionally or alternatively, the collected physiological data may be considered during designing the final image of the electronic tattoo.

In aspects, the target location may be obtained via user-input on a desired location. Additionally or alternatively, the target location may be obtained a look-up table based on the desired function.

In aspects, physiological data may be collected based on the desired function.

In aspects, the physiological data may include one or more or dimensional or topographical information of a surface of the target location such as area, size and/or curvature, mechanical properties of the surface of the target location, locations of relevant physiological sensor points, such as pulse points, muscle locations and/or stimulation points.

In aspects, the physiological data may be collected via a device, particularly a user device, including one or more of a camera, a time of flight sensor, acoustic and/or ultrasonic sensing capabilities.

In aspects, collecting physiological data may include instructing a user to acquire the physiological data.

The present disclosure further relates to a storage media. The storage media comprises instructions that when executed are adapted to cause a computing device to perform the method of any one of the preceding aspects.

The present disclosure further relates to a computing device. The computing device is configured to perform the method of any one of the preceding aspects.

The present disclosure further relates to a tattoo design system for designing a user-specific electronic tattoo. The tattoo design system comprises a storage media and a computing device. The storage media comprises instructions that when executed are adapted to cause a computing device to perform the method of any one of the preceding aspects. The computing device is configured to perform the method of any one of the preceding aspects.

In aspects, the tattoo design system may further comprise a component database. The component database may store entries on the various models, shapes and variants of tattoo components that are available to the user to purchase, print or produce. In examples, the component database may store visual and/or aesthetic features, functions, constraints and/or further necessary information of circuit components such as power requirements, resistance, voltage, current properties, component connection type and/or component size.

In aspects, the tattoo design system may further comprise a tattoo image database. The tattoo image database may store existing tattoo designs. Existing tattoo designs may comprise pre-defined examples (e.g. default examples) or uploaded user-created designs. Additionally or alternatively, salient features, e.g. visual and/or aesthetic features, may be stored on the tattoo image database.

In aspects, the tattoo design system may be connectable to a user-interface for obtaining user-input on one or more a desired location, one or more desired functions and a desired appearance of the electronic tattoo, for communicating a final image of the electronic tattoo to a user, and/or for communicating user instructions to a user.

In aspects, the tattoo design system may be connectable to a physiological data collection system for collecting physiological data on a target location of the electronic tattoo.

It has been found an approach to combine on-skin electronics with AI-enabled circuit design and machine learning for creative applications to design modular on-skin electronic components in such a way as to balance circuit functionality with the user's desired aesthetic appearance.

A method for designing user-specific electronic tattoos with customized appearance and function is described. The method may enable users to produce electronic tattoos that are both visually appealing and functional. That means, the disclosed concept is designed to enable unskilled users to create functional and aesthetically attractive electronic tattoos. Users without experience in electronics may be able to create wearable circuits from modular components that function effectively. Users may be able to design tattoos that incorporate these circuits unobtrusively into aesthetically pleasing and personalized designs. Less creative users may be able to make electronic tattoo designs that are visually attractive and represent their personal style. In other words, an electronic tattoo design is created in such a way that it incorporates all the necessary electronic components to achieve a user-specified function, while having a visual style and appearance that matches the user's aesthetic goals. Therefore, the method suggests the user to apply specific tattoo components in a specific arrangement in order to produce a specific design of an electronic tattoo. The tattoo components may include electronic components, specifically flexible electronic components, conductive inks, non-conductive inks, and, in examples, a base layer.

### Description of the Drawings

Other characteristics will be apparent from the accompanying drawings, which form a part of this disclosure. The drawings are intended to further explain the present disclosure and to enable a person skilled in the art to practice it. However, the drawings are intended as nonlimiting examples. Common reference numerals on different figures indicate like or similar features.
- **FIG. 1**: shows a schematic view of the tattoo design system;
- **FIG. 2**: shows a flow chart of the computer-implemented method carried out by the tattoo design system;
- **FIG. 3**: shows general method steps of the computer-implemented method;
- **FIG. 4**: shows method steps of an example configuration of the computer-implemented method;
- **FIG. 5**: shows an illustrative example application of the computer-implemented method.

### Detailed Description

Embodiments of the computer-implemented method and the tattoo design system according to the disclosure will be described with reference to the figures as follows.

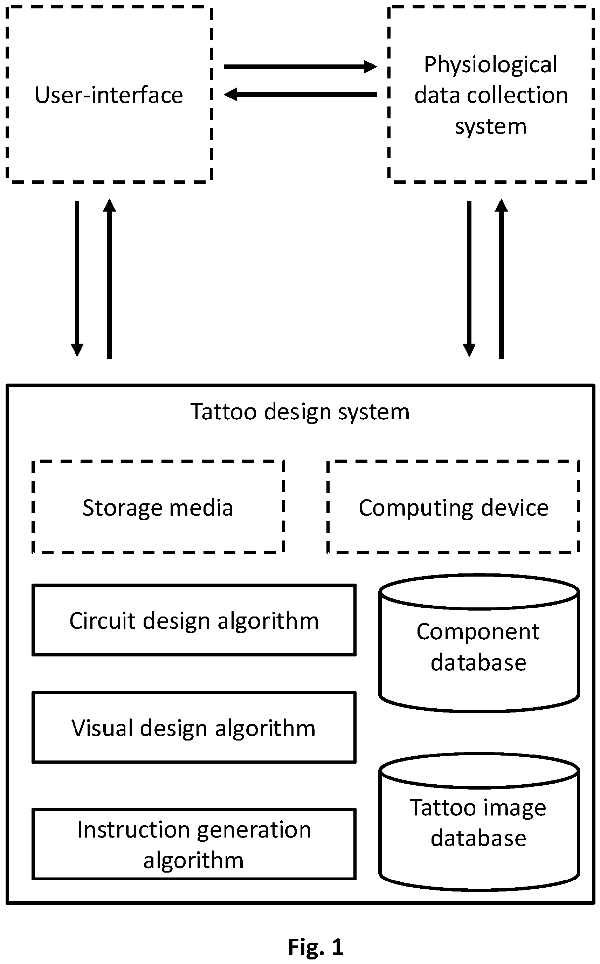

**Fig. 1** is a schematic view of a tattoo design system for designing a user-specific electronic tattoo interacting with a user-interface and a physiological data collection system. In other words the tattoo design system is configured to interact with a user-interface and a physiological data collection system. The tattoo design system comprises a storage media and a computing device. The storage media comprises instructions that when executed are adapted to cause the computing device to perform the presently disclosed computer-implemented method. In other words, the computing device is configured to perform the computer-implemented method. In examples, the tattoo design system is configured to perform the computer-implemented method. In some embodiments, the storage media may be a separate device and may comprise instructions that when executed are adapted to cause a computing device to perform the computer-implemented method as described herein.

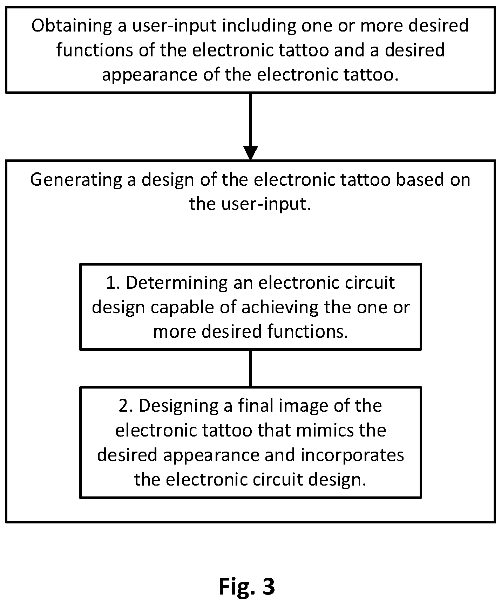

With reference to **Fig. 3****,** the basic method steps of the computer-implemented method for designing a user-specific electronic tattoo are shown. In a first step, a user-input input is obtained. The user-input includes one or more desired functions of the electronic tattoo and a desired appearance of the electronic tattoo. In a next step, a design of the electronic tattoo is generated based on the user-input. The generation of the design of the electronic tattoo includes determining an electronic circuit design which is capable of achieving the one or more desired functions. When the electronic circuit design is determined, a final image of the electronic tattoo is designed that mimics the desired appearance and incorporates the electronic circuit design. The presently disclosed method can enable a user, particularly an unskilled user, to produce electronic tattoos that are both visually appealing and functional.

Designing a final image of the electronic tattoo can be understood as creating an electronic tattoo design which then may then be produced by a user. The desired functions may include non-medical and medical applications. For instance, the desired functions may include one or more physiological sensors, e.g. heart rate sensor, sweat sensor, or any other, one or more visual displays, one or more haptic or other feedback system, or any other function known in the art. The one or more desired functions may be selected from a pre-set list of possible functions. In examples, the one or more desired functions may be created by a user.

When performing the method, various algorithms such as a circuit design algorithm, a visual design algorithm and, if user instructions are generated, a instruction generation algorithm are applied. The algorithms may be part of the tattoo design system (see, **Fig. 1**). Furthermore, the tattoo design system comprises a component database and a tattoo image database. The component database stores information such as entries on the various models, shapes and variants of tattoo components, particularly circuit components, that are available to the user to purchase, print or produce. The user may provide the system with information on which circuit components are available to him/her. In examples, the component database may store visual and/or aesthetic features, functions, constraints and/or further necessary information of circuit components such as power requirements, resistance, voltage, current properties, component connection type and/or component size. The tattoo image database stores existing tattoo designs. Existing tattoo designs may be pre-defined examples (e.g. default examples) or uploaded user-created designs. This enables a user to select from the existing tattoo designs to provide the desired appearance. The tattoo image database may further store salient features, e.g. visual and/or aesthetic features of the respective tattoo designs. This enables the system or method to directly use the known visual and/or aesthetic features for the further process of designing a user-specific electronic tattoo. In some embodiments, the tattoo design system does not comprise a tattoo image database.

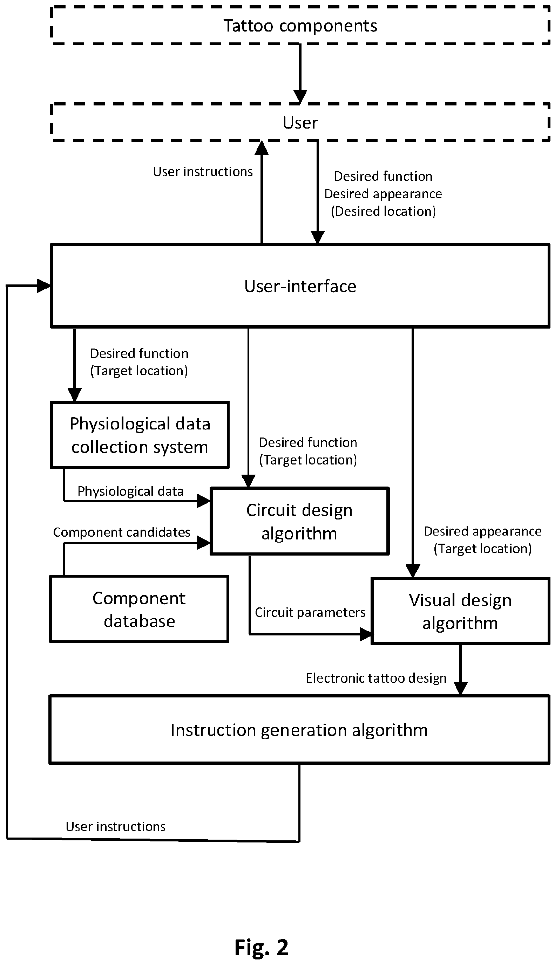

As schematically depicted in **Fig. 1** (see, also **Fig. 2**), the tattoo design system is connectable to a user-interface. In other words, the tattoo design system is configured to communicate with a user-interface. In examples, the tattoo design system is configured to be connected to a user-interface. Connection in this regard may be a wired or wireless data connection. In some embodiments, the user-interface may be part of the tattoo design system. Via the user-interface user-input on one or more of a desired location, a desired function and a desired appearance of the electronic tattoo can be obtained. It may also be possible that more than one desired function is provided via the user-interface. The final image of the electronic tattoo can be communicated to a user via the user-interface. Furthermore, user instructions can be communicated to a user via the user-interface.

The physiological data collection system may be part of the tattoo design system. In examples, the physiological data collection system may be a separate device and the tattoo design system may simply communicate, i.e. may be configured to communicate, with the physiological data collection system (see, **Fig. 1**). In examples, the tattoo design system may be connectable to the physiological data collection system. Via the physiological data collection system physiological data on a target location of the electronic tattoo can be collected. In other words, the tattoo design system is configured to obtain (or collect) physiological data via the physiological data collection system.

With regard to **Fig. 2****,** the method and system of the present disclosure is described in more detail. User-input is provided by a user via a user-interface. As mentioned above, the user-input includes at least one or more desired functions and a desired appearance. In examples, the user-input may further include a desired location. Desired location can be understood as a location where the user wishes to apply the electronic tattoo to. In some embodiments, desired location can mean a location on a user's skin where the user wishes to apply the electronic tattoo. It should be understood that the provision of a desired location by the user is needed depending on circumstances. In some embodiments a target location may be provided by the system. In embodiments, the location of the application of the electronic tattoo may be less relevant. A target location may mainly depend on the functions the electronic tattoo is desired to fulfill. Thus, either no target location may be necessary or desired, a target location may be provided by a user that may then be referred to it as "desired location", for instance because it is desired to place the electronic tattoo at a specific location, i.e. desired location, or a target location is provided by the system because of specific requirements of one or more desired functions. In the last case, the system may suggest an optimum location for the electronic tattoo based on the desired function. For instance, an electronic tattoo with heart rate sensor as the desired function should be placed at a location where the heart rate can be measured. Such locations may be stored in and accessed from a lookup table that has been pre-populated with known good locations for specific sensor placement. In examples, it may be possible that a desired location is provided and that a target location is required due to one or more functions. In that case a suitable target location may be suggested to the user. If more target locations are available, it may be compared if one of the required target locations matches with the desired location. Although the target location described herein is on-skin, i.e. on a skin surface of a user, in some embodiments, the electronic tattoo may be applied to other target locations. For instance, the electronic tattoo may be placed on-skin of an animal or off-skin. Off-skin applications, may for instance comprise an application to a furniture surface, a building surface, a textile surface (e.g. apparel surface), or any other surface (e.g. natural surface). In general, on-skin can be understood as on a skin surface of the user's body on which the tattoo should be placed. Although, in general terms it will be only spoken of a user or the user, it should be understood that a first user can for instance prepare, design and apply the electronic tattoo to another user, i.e. to the skin of the other user. In some embodiments, it may also be possible to select between one or more possible target locations. In some embodiments, more than one possible target location may be provided or obtained by the user or the method/system.

Thus, if a target location is desired and/or required, the computer-implemented method obtains a target location for application of the electronic tattoo. Physiological data on the target location is collected and the physiological data may then be considered during the further method steps of determining the electronic circuit design and designing the final image of the electronic tattoo. The physiological data may be collected based on the desired function. In other words, if and/or which physiological data is collected is decided based on the desired function. For instance, some desired functions require specific locations, e.g. detecting a pulse point or heart rate monitoring, whilst others, e.g. a sweat sensor does not necessarily require a specific location.

The physiological data is collected via the physiological data collection system. The physiological data collection system may include one or more of a camera, a time of flight (ToF) sensor, acoustic and/or ultrasonic sensing capabilities to collect the physiological data. The physiological data collection system may be comprised in a user device or may be a separate device, e.g. a completely separate device or integrated in the tattoo design system. In other words, the physiological data may be collected via a device, particularly a user device, including one or more of a camera, a time of flight sensor, acoustic and/or ultrasonic sensing capabilities. The user device may be a smartphone. Additionally or alternatively, the user device may use the capabilities of the device to gather the physiological data. The physiological data may gathered through components of the user device including a camera, a time of flight sensor, acoustic and/or ultrasonic sensing capabilities. The physiological data may include one or more or of dimensional or topographical information of a surface of the target location such as area, size and/or curvature, mechanical properties of the surface of the target location, tissue properties of the surface of the target location, e.g. when the target location is a skin surface, i.e. a target skin surface, locations of relevant physiological sensor points, such as pulse points, muscle locations and/or stimulation points. The type of physiological data which is collected may depend on the one or more desired functions.

Instructions may be provided to collect the physiological data. In other words, instructions may be provided on how and which physiological data is to be collected. In examples, instructions for collection physiological data may be provided to the user. Instruction the user may include guiding a user to acquire the physiological data via the user-interface. Instructing the user may include requesting a specific capability to be used to collect a specific type of physiological data. Additionally or alternatively, instructing the user may include requesting a specific area to be scanned. For instance, where the desired function is a heart rate sensor, the physiological data collection system may request that an appropriate sensor, e.g. IR camera, photoplethysmography is used to identify optimum placement of the sensor. The physiological data collection system may further request the user to scan the area of the chest. For instance, where the desired function is a display, the physiological data collection system may request that an appropriate sensor, e.g. camera, ToF sensor, is used to identify a sufficiently large surface area for the display component relative to its known dimensions. In some embodiments, physiological data may be collected automatically.

The desired appearance may be provided by uploading an image file. In some embodiments, the desired appearance may be provided by selecting an image file from the tattoo image database which stores existing tattoo designs. Existing tattoo designs may comprise pre-defined examples, e.g. default examples, or uploaded user-created designs. Additionally or alternatively, salient features, e.g. visual and/or aesthetic features, of the image file may be identified and extracted by image recognition. In examples, the visual and/or aesthetic features may be conveyed into a target image. By extracting salient features and/or key features, e.g. visual and/or aesthetic features, of the image file a conversion into a form to recreate a tattoo may be possible. In some embodiments, the desired appearance may be provided by uploading basic preferences, particularly user preferences. The preferences may regard one or more of shape, size, colouration, style, visual features and/or aesthetic features. In examples, the preferences, particularly the visual and/or aesthetic features, may be conveyed into a target image. For instance, the desired appearance may be provided by uploading a description, e.g. a textual or audio description of the desired appearance. Salient features of basic preferences, particularly visual and/or aesthetic features, are identified and extracted by semantic recognition.

The user-input on the desired appearance may include constraints on the desired appearance. The constraints may define a degree of how close the desired appearance is to be met. For instance, constraint options may include closest possible match or iterative match. In other words, the user may select one of two options of closest possible match or iterative match. For instance, the user may indicate that an uploaded image should be reproduced as closely as possible, i.e. closest possible match. In this case the tattoo components, particularly circuit components and electrical connections, would be incorporated into the known, fixed parameters of the image. For the case of iterative match, the user may, for instance, indicate that the uploaded image is simply an example from which a specific style or feature should be used, or may provide simple textual instructions describing the preferences. In this case the tattoo components, particularly circuit components and electrical connections, would be integrated into the image in a more iterative manner, whereby the image itself may be varied or modified to match with the components more effectively.

After collecting physiological data (if applicable), the electronic circuit design is determined. Therefore, the circuit design algorithm is applied (see, **Fig. 2**). The circuit design algorithm generates circuit parameters which are necessary for achieving the one or more desired functions. Circuit parameters can be understood as a set of requirements which need to be fulfilled to achieve the one or more desired functions. In other words, circuit parameters may describe how a functioning circuit should be assembled to meet the desired function requirement. Generating circuit parameters includes identifying circuit components which fulfill the desired functions, i.e. circuit component candidates or suitable circuit components. Circuit components can be understood as functional circuit components, e.g. a sensor, a power source, an electrode or any other electrical component known in the art. Circuit component candidates can be understood as circuit components which could be used to build the electronic circuit. Identifying circuit component candidates may include determining circuit component candidates from circuit components which are stored in the component database and which fulfill the desired functions. Determining circuit component candidates may include obtaining information from the component database regarding one or more of power requirements, resistance, voltage, current properties, circuit component connection type, and/or circuit component size. Furthermore, electrical connections between the circuit component candidates are identified, e.g. electrical connections which are required by the circuit component candidates. This may include identifying an order of connection between circuit components. Furthermore, a specific conductive ink to be use for producing the electrical connections may be determined. In examples, constraints such as circuit layout, circuit component separation, electrical connection length and/or required circuit component location may be identified. The required circuit component location may be dictated for instance by the desired function or the desired location - e.g. pulse points or other physiological data features. Thereby, physiological data may be considered, for instance, when determining required circuit component location and/or electrical connection length. When performing these method steps, the circuit design algorithm may apply computational circuit design techniques.

In some embodiments, optimum circuit component candidates may be pre-selected from the circuit component candidates based on predefined selection metrics. The predefined selection metrics for selecting the optimum circuit component candidates may include one or more of dimensional fit to the desired or determined location, efficiency and/or performance of the electronic circuit, availability of circuit components, and price of circuit components. Dimensional fit can be understood as dimensional fit to the user's body as indicated by the physiological data. Availability of circuit components may be dictated based on, e.g. the user's geographical location, personal skills, electronic tattoo component resources, e.g. circuit components, conductive inks and/or non-conductive inks, and/or technological infrastructure available to the user, e.g. printers. Availability may include the ability to print or produce at site, e.g. ability to print at home, or to purchase the electronic tattoo components. The price may be pre-set by the user. Additional other user preferences may also be used as selection metrics.

Selecting the optimum circuit component candidates may be prioritized by one or more of the predefined selection metrics. Particularly, selecting the optimum circuit component candidates may be prioritized by an order of two or more of predefined selection metrics. For instance, selecting the optimum circuit component candidates may be prioritized by first availability, second best dimensional fit and third price. In examples, it may be prioritized by first, ensuring that only circuit components the user can access are used in the circuit design. Second, by ensuring good function of the circuit within the dimensional constraints of the desired location using the available circuit components. Third, by meeting the user's price or other preferences.

After determining the electronic circuit design, the final image of the electronic tattoo is designed. Therefore, the visual design algorithm is applied (see, **Fig. 2**). The visual design algorithm obtains the previously generated circuit parameters. Then a target image of the electronic tattoo is created. Creating the target image includes determining visual and/or aesthetic features of the desired appearance. Then the circuit parameters are incorporated into the target image. Then the final image of the electronic tattoo may be created by filling out part of or all remaining sections of the target image with non-conductive tattoo component ink. Remaining sections can be understood as those sections of the target image which have not yet been filled out by tattoo components for implementing the circuit parameters, i.e. circuit components and electrical connections, e.g. conductive inks. Non-conductive tattoo component ink, also referred to as non-conductive ink, may include various ink types and/or colours which are available to the user.

In general, the electronic tattoo may consist of various electronic tattoo components, also referred to as electronic tattoo component resources. These electronic tattoo components may include one or more of circuit components, also referred to as electronic tattoo component devices, conductive inks, also referred to as conductive tattoo component ink, non-conductive inks, also referred to as non-conductive tattoo component ink and a base layer. In examples, the electronic tattoo components comprise at least a circuit component and conductive inks. The base layer may be a plastic or organic substrate which may be known in the art and which may be applied to a user's skin or to another application surface.

Incorporating the circuit parameters into the target image includes selecting final circuit components. That means, final circuit components are selected from the previously identified circuit component candidates, i.e. optimum circuit component candidates and/or circuit component candidates. Selecting final circuit components includes comparing visual and/or aesthetic features of the circuit component candidates, e.g. optimum circuit component candidates and/or circuit component candidates, with the visual and/or aesthetic features of the desired appearance. The visual and/or aesthetic features of the circuit component candidates may include one or more of colour, shape and/or size of the circuit component candidates. The visual and/or aesthetic features of the circuit component candidates may be obtained from the component database based on the circuit parameters. Determining the visual and/or aesthetic features of the desired appearance may include applying machine vision techniques analyzing aesthetic/artistic style, colour palette and/or image features of an image file provided by the user as user-input on the desired appearance. In some embodiments, determining the visual and/or aesthetic features of the desired appearance may include applying semantics analysis of a non-image based user-input, such as textual input, on the desired appearance.

In some embodiments, selecting final circuit components may further include selecting those circuit components from the circuit component candidates which closest match the visual and/or aesthetic features of the desired appearance based on the comparison. For instance, in embodiments where one desired function requires three different circuit components (each fulfilling a required subfunction), the three closest matching components (which fulfill the three required subfunctions) are selected from the circuit component candidates. In other examples only one final circuit component may be required to fulfill the desired one or more functions. In that case it should be understood that only one final circuit component is selected. That means only that circuit component is selected from the circuit component candidates which closest matches the visual and/or aesthetic features of the desired appearance based on the comparison. In some embodiments, sections of the target image may be altered to match the visual and/or aesthetic features of those circuit components for which no circuit component candidates which match the visual and/or aesthetic features of the desired appearance are present. The comparison may be applied to identify opportunities for integration of the shapes, colourations and sizes of the circuit components in an appropriate and pleasing way to fulfill the user's desired appearance. Circuit component candidate selection may be varied to match the aesthetic properties of the image when components with the same function but different shape, size or colouration are available that more closely match the image requirements. The extent of altering sections may also be dependent on a user's constraints regarding the desired appearance, e.g. closest possible match or iterative match.

Incorporating the circuit parameters into the target image includes placing the final circuit components into the target image. That means, certain aesthetic and/or visual features of the target image are fully or partly replaced by the final circuit parameters. In other words, the final circuit parameters are conveyed into certain aesthetic and/or visual features of the target image, particularly based on the comparison. The final circuit components may be defined as electronic tattoo component devices. The identified electrical connections may be placed into the target image. That means, certain aesthetic and/or visual features of the target image are fully or partly replaced by the conductive ink. In other words, the electrical connections are conveyed into certain aesthetic and/or visual features of the target image. In case there are no adequate aesthetic and/or visual features of the target image being replaced by conductive ink, the target image is altered. The identified electrical connections may be defined as conductive tattoo component ink. When incorporating the electrical connections, the target image may have an impact on routing of the conductive ink and/or choice of the conductive ink. In some embodiments, wherein a specific electrical resistance between two circuit components is needed, an appropriate length of the routing of conductive ink and/or a specific conductive ink are chosen to meet the resistance requirements of the circuit components.

In some embodiments, the user can adjust or edit the aesthetic and/or visual features of the desired appearance at any stage during the method. In some embodiments, the user can adjust or edit the desired location at any stage during the method. Particularly, the functional and/or aesthetic features of the target image and/or the functional and/or aesthetic features of the final image may be adjusted by a user. In some aspects, the user may adjust or edit the desired functions of the electronic tattoo at any stage during the method. In some aspects, the user may be informed of impacts and/or improvements, e.g. improvements to functionality and/or aesthetics, resulting from the user adjustment. For instance, the user may be informed when he/she moves the heart rate sensor to somewhere functionally less optimal to improve the aesthetic design. In some embodiments, the user may be informed during or after conducting adjustments.

In some embodiments, the user-specific electronic tattoo is a temporary tattoo. In other embodiments, the user-specific electronic tattoo is a permanent tattoo. Depending on the material (e.g. ink), the user-specific electronic tattoo is either temporary or permanent. For instance, a temporary tattoo could be designed to be temporarily placed but can be removed and placed back completely on by the user. In some embodiments, the user-specific electronic tattoo comprises circuit components which are reusable but wherein components like conductive tattoo component ink and/or non-conductive tattoo component ink are temporarily. For instance, the design of temporary inks may be changed/adapted before each reuse of the user-specific electronic tattoo, e.g. the reuse of the circuit components.

In some embodiments, user instructions for producing the electronic tattoo are generated. The user instructions may be generated after designing the final image of the electronic tattoo. In other words, user instructions for producing the electronic tattoo are generated based on the final image of the electronic tattoo. Therefore, the instructions generation algorithm is applied (see, **Fig. 2**). The instructions generation algorithm may generate one or more of various different types of user instructions. For instance, a text-based list of instructions in natural language may be generated. In examples, text generation machine learning techniques may be user to generate the text-based list. Additionally or alternatively to the text-based list, a sequence of step-by-step images of the electronic tattoo may be generated. Additionally or alternatively, the final image of the electronic tattoo may be projected at a desired location. In some aspects, the projection may include applying augmented reality technologies overlay or similar technologies. The user instructions are communicated to the user. In some embodiments, the user may be provided with a list of electronic tattoo components required to produce the electronic tattoo. The electronic tattoo components may comprise one or more of circuit components, conductive inks, non-conductive inks and a base layer. In examples, the list of electronic tattoo components comprises at least circuit components and conductive inks. In some embodiments, electronic tattoo components may be purchased via the user-interface of the tattoo design system. Producing the electronic tattoo based on the final image may include producing the electronic tattoo on a user's skin or pre-producing the electronic tattoo for application of the electronic tattoo on a skin or any other surface. The user instructions may be communicated to the user acoustically. In some embodiments, the user instructions may, additionally or alternatively, be communicated to the user visually. In some embodiments, communicating the user instructions includes projecting an image of the electronic tattoo at a desired location. In some aspects, the projection may include applying augmented reality technologies overlay or similar technologies.

After generating and communicating the user instructions, the user receives the user instructions, acquires the necessary tattoo components and creates the electronic tattoo, for instance on their body at the desired location (or required target location).

In some embodiment, alternatively or additionally to user instructions, the final image of the electronic tattoo may be provided, e.g. visualized, via the user-interface. Additionally or alternatively, user instructions for creation of the electronic tattoo may be provided, e.g. visualized, via the user-interface. Additionally or alternatively, audio instructions may be given to the user.

### Example method

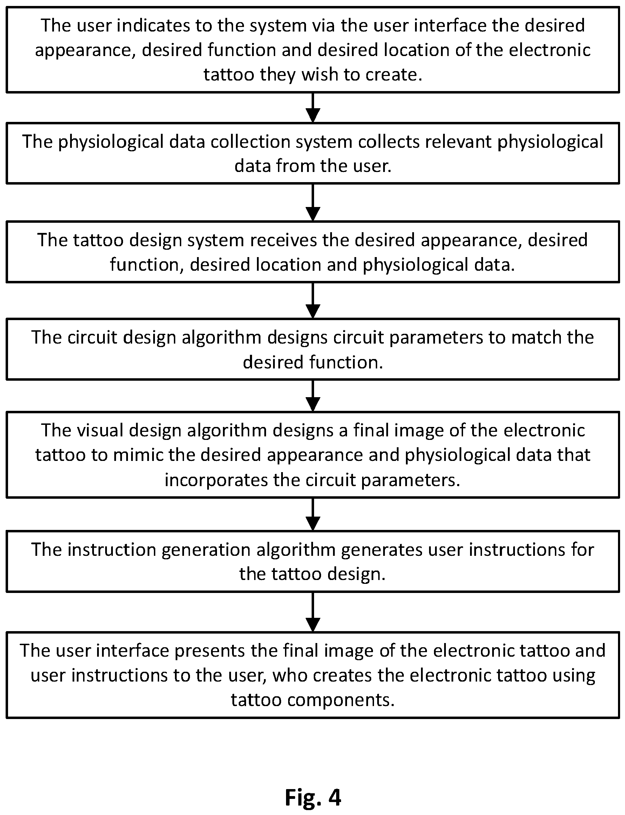

An example method of carrying out the present disclosure is shown in **Fig. 4****.** In a first step, the user indicates to the system via the user interface the desired appearance, desired function and, in examples desired location of the electronic tattoo they wish to create. In examples, the physiological data collection system then collects relevant physiological data from the user. In examples, the tattoo design system then receives the desired appearance and desired function. In examples, the tattoo design system also receives the desired location. In examples, the tattoo design system also receives the physiological data. Then, the circuit design algorithm designs circuit parameters to match the desired function. Then, the visual design algorithm designs a final image of the electronic tattoo to mimic the desired appearance and physiological data that incorporates the circuit parameters. Then, the instruction generation algorithm generates user instructions for the tattoo design. Then, the user interface presents the final image of the electronic tattoo and user instructions to the user, who creates the electronic tattoo using tattoo components.

### Example application

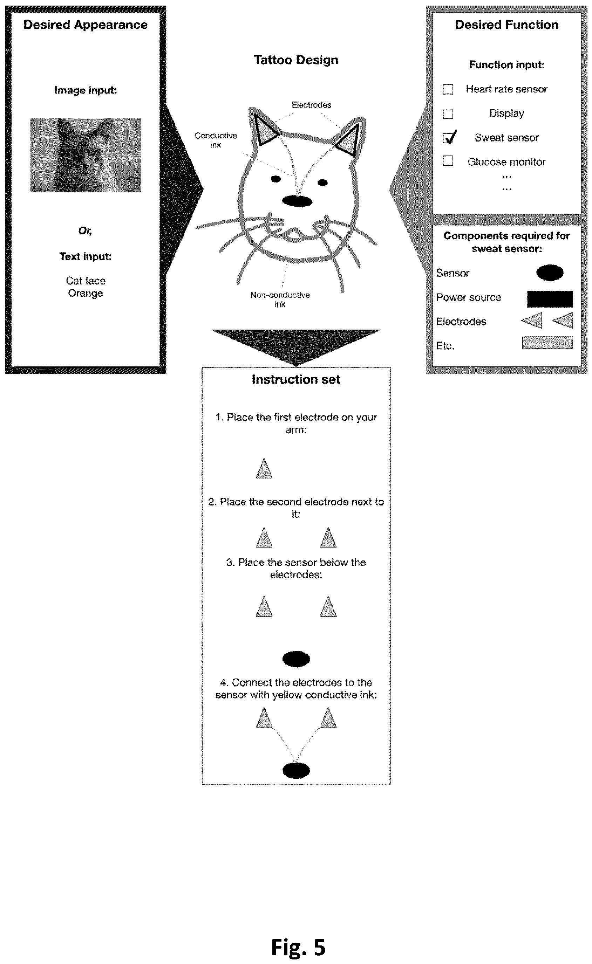

An illustrative application of carrying out the presently disclosed computer-implemented method is exemplary depicted in **Fig. 5****.** In the center of **Fig. 5** is shown the tattoo design, i.e. the generated design of the electronic tattoo. In other words, this is the final image of the electronic tattoo, which is designed according to the presently disclosed method. The tattoo design comprises various electronic tattoo components which are incorporated into the target image (not shown). In this example, user-input includes a desired appearance in the form of an uploaded image of a cat. In examples, basic preferences are given as user-input describing the desired appearance by providing a textual description - in this example an orange cat face. In the latter example key features of the desired appearance may be determined by semantic analysis. In this case key features are, for instance aesthetic and visual features which are characteristic for an orange cat face, e.g. orange colour, characteristic ears, eyes, nose mouth and whiskers. In the first example of uploading a cat image, also key features of the desired appearance may be determined but in this case by image recognition. Also in the first case, similar aesthetic and visual features which are characteristic for the uploaded image - an orange cat face, e.g. orange colour, characteristic ears, eyes, nose mouth and whiskers, may be determined. Based on the determined aesthetic and visual features, a target image (not shown) is created. In the present example a sweat sensor is selected as desired function. The circuit design algorithm generates circuit parameters which are necessary for achieving the function of a sweat sensor. That includes identifying circuit component candidates, e.g. a sensor, a power source two electrodes. Furthermore, that includes identifying necessary electrical connections, e.g. between the sensor and the electrodes, and identifying constraints, e.g. arranging a respective power source adjacent a respective electrode. In the next step, the visual design algorithm merges the circuit parameters with the target image. Thereby, the aesthetic and visual features of the desired appearance are compared with the aesthetic and visual features of the tattoo components as defined in the circuit parameters. For instance, the comparison leads to the result that the ears of the cat have a certain similarity with the electrodes. Then the electrodes will be integrated or conveyed in the ears of the target image. This comparison is done for each tattoo component of the circuit parameters until all necessary circuit components and electrical connections are incorporated in the target image. Then the remaining sections, i.e. the remaining aesthetic and visual features, e.g. the mouth, the whiskers and the basic head shape, of the target image will be filled out with non-conductive ink to complete the final image of the electronic tattoo. This final image of the electronic tattoo may then be used by a user to create the electronic tattoo in a physical form. Therefore, user instructions may be generated. In the present example, the user instructions comprise a text-based list including step-by-step images. For illustrative purposes only the first four steps of the user instructions are shown.

It should be understood that the present invention can also (alternatively) be defined in accordance with the following embodiments:
1. A computer-implemented method for designing a user-specific electronic tattoo comprising:
   - obtaining a user-input including one or more desired functions of the electronic tattoo and a desired appearance of the electronic tattoo,
   - generating a design of the electronic tattoo based on the user-input including:
      ∘ determining an electronic circuit design capable of achieving the one or more desired functions, and
      ∘ designing a final image of the electronic tattoo that mimics the desired appearance and incorporates the electronic circuit design.
2. The computer-implemented method of embodiment 1, wherein the user-input is provided by a user via a user-interface.
3. The computer-implemented method of embodiment 2, wherein the final image of the electronic tattoo and/or user instructions for creation of the electronic tattoo are provided, specifically visualized, via the user-interface.
4. The computer-implemented method of any one of embodiments 1 to 3, wherein the one or more desired functions are selected from a pre-set list of possible functions.
5. The computer-implemented method of any one of embodiments 1 to 4, wherein the desired appearance is provided by uploading an image file.
6. The computer-implemented method of any one of embodiments 1 to 5, wherein the desired appearance is provided by selecting an image file from a tattoo image database which stores existing tattoo designs.
7. The computer-implemented method of any one of embodiments 5 or 6, wherein salient features, specifically visual and/or aesthetic features, of the image file are identified and extracted by image recognition, and particularly, wherein the visual and/or aesthetic features are conveyed into a target image.
8. The computer-implemented method of any one of embodiments 1 to 7, wherein the desired appearance is provided by uploading basic preferences regarding one or more of shape, size, colouration, style, visual features and/or aesthetic features, and particularly,
   wherein the visual and/or aesthetic features are conveyed into a target image.
9. The computer-implemented method of any one of embodiments 1 to 8, wherein the user-input on the desired appearance includes constraints on the desired appearance defining a degree of how close the desired appearance is to be met.
10. The computer-implemented method of any one of the preceding embodiments, wherein determining the electronic circuit design comprises applying a circuit design algorithm configured to generate circuit parameters for achieving the one or more desired functions.
11. The computer-implemented method of embodiment 10, wherein generating circuit parameters includes:
   - identifying circuit component candidates which fulfill the desired functions,
   - identifying electrical connections between circuit component candidates, and
   - identifying constraints such as circuit layout, circuit component separation, electrical connection length and/or required circuit component location.
12. The computer-implemented method of embodiment 11, wherein identifying circuit component candidates includes:
   - determining circuit component candidates from circuit components stored in a component database which fulfill the desired functions, and particularly,
   - pre-selecting from the circuit component candidates the optimum circuit component candidates based on predefined selection metrics.
13. The computer-implemented method of embodiment 12, wherein the predefined selection metrics for selecting the optimum circuit components include one or more of:
   - dimensional fit to the desired or determined location,
   - efficiency and/or performance of the electronic circuit,
   - availability of circuit components, and
   - price of circuit components.
14. The computer-implemented method of embodiment 13, wherein selecting the optimum circuit components is prioritized by one or more of the predefined selection metrics, and particularly, by an order of two or more of predefined selection metrics.
15. The computer-implemented method of any one of embodiments 11 to 14, wherein determining circuit component candidates includes obtaining information from the component database regarding one or more of power requirements, resistance, voltage, current properties, circuit component connection type, and/or circuit component size.
16. The computer-implemented method of any one of embodiments 10 to 15, wherein designing the final image of the electronic tattoo comprises applying a visual design algorithm configured to:
   - obtain the previously generated circuit parameters,
   - create a target image which includes determining visual and/or aesthetic features of the desired appearance,
   - incorporate the circuit parameters into the target image, and
   - create the final image of the electronic tattoo by filling out part of or all remaining sections of the target image with non-conductive tattoo component ink.
17. The computer-implemented method of embodiment 16, wherein incorporating the circuit parameters into the target image includes:
   - selecting final circuit components, placing them into the target image, and particularly, defining them as electronic tattoo component devices,
   - placing the identified electrical connections into the target image, and particularly, defining them as conductive tattoo component ink.
18. The computer-implemented method of embodiment 17, wherein selecting final circuit components includes:
   - comparing visual and/or aesthetic features of the circuit component candidates with the visual and/or aesthetic features of the desired appearance,
   - selecting those circuit components from the circuit component candidates which closest match the visual and/or aesthetic features of the desired appearance based on the comparison, and
   - altering sections of the target image to match the visual and/or aesthetic features of those circuit components for which no circuit component candidates are present which match the visual and/or aesthetic features of the desired appearance.
19. The computer-implemented method of embodiment 18, wherein the visual and/or aesthetic features of the circuit component candidates include one or more of colour, shape and/or size of the circuit component candidates, and particularly,
   wherein the visual and/or aesthetic features of the circuit component candidates are obtained from the component database based on the circuit parameters.
20. The computer-implemented method of any one of embodiments 16 to 19, wherein determining the visual and/or aesthetic features of the desired appearance includes one or more of:
   - applying machine vision techniques analyzing aesthetic/artistic style, colour palette and/or image features of an image file provided by the user as user-input on the desired appearance,
   - applying semantics analysis of a non-image based user-input, such as textual input, on the desired appearance.
21. The computer-implemented method of any one of embodiments 16 to 20, wherein at any stage during the method, a user can adjust or edit the aesthetic and/or visual features of the desired appearance.
22. The computer-implemented method of any one of the preceding embodiments, further comprising:
   - generating user instructions for producing the electronic tattoo based on the final image of the electronic tattoo,
   - communicating the user instructions to the user, and
   - providing the user with a list of electronic tattoo components required to produce the electronic tattoo.
23. The computer-implemented method of embodiment 22, wherein generating user instructions includes applying an instructions generation algorithm configured to:
   - generate a text-based list of instructions in natural language, specifically using text generation machine learning techniques, and/or
   - generate a sequence of step-by-step images of the electronic tattoo, and/or
   - project the final image of the electronic tattoo at a desired location.
24. The computer-implemented method of any one of embodiments 22 or 23, wherein the user instructions are communicated to the user acoustically and/or visually, and particularly,
   wherein communicating the user instructions includes projecting an image of the electronic tattoo at a desired location.
25. The computer-implemented method of any one of embodiments 22 to 24, wherein the electronic tattoo components comprise:
   - circuit components,
   - conductive inks,
   - non-conductive inks, and particularly,
   - a base layer.
26. The computer-implemented method of any one of the preceding embodiments further comprising:
   - obtaining a target location for application of the electronic tattoo,
   - collecting physiological data on the target location,
   - considering the collected physiological data during determining the electronic circuit design and/or during designing the final image of the electronic tattoo.
27. The computer-implemented method of embodiment 26, wherein the target location is obtained via user-input on a desired location and/or, wherein the target location is obtained from a look-up table based on the desired function.
28. The computer-implemented method of any one of embodiments 26 or 27, wherein physiological data is collected based on the desired function.
29. The computer-implemented method of any one of embodiments 26 to 28, wherein the physiological data includes one or more or:
   - dimensional or topographical information of a surface of the target location such as area, size and/or curvature,
   - mechanical of the surface of the target location,
   - locations of relevant physiological sensor points, such as pulse points, muscle locations and/or stimulation points.
30. The computer-implemented method of any one of embodiments 26 to 29, wherein the physiological data is collected via a device, particularly a user device, including one or more of:
   - a camera,
   - a time of flight sensor,
   - acoustic and/or ultrasonic sensing capabilities.
31. The computer-implemented method of any one of embodiments 26 to 30, wherein collecting physiological data includes instructing a user to acquire the physiological data.
32. A storage media comprising instructions that when executed are adapted to cause a computing device to perform the method steps of any one of the preceding embodiments.
33. A computing device configured to perform the method steps of any one of the preceding embodiments.
34. A tattoo design system for designing a user-specific electronic tattoo comprising the storage media of embodiment 32 and the computing device of embodiment 33.
35. The tattoo design system of embodiment 34 further comprising a component database and a tattoo image database.
36. The tattoo design system of any one of embodiment 34 or 35 being connectable to a user-interface:
   - for obtaining user-input on one or more a desired location, one or more desired functions and a desired appearance of the electronic tattoo,
   - for communicating a final image of the electronic tattoo to a user, and/or
   - for communicating user instructions to a user.
37. The tattoo design system of any one of embodiment 34 to 36 being connectable to a physiological data collection system for collecting physiological data on a target location of the electronic tattoo.

## Claims

1. A computer-implemented method for designing a user-specific electronic tattoo comprising:
- obtaining a user-input including one or more desired functions of the electronic tattoo and a desired appearance of the electronic tattoo,
- generating a design of the electronic tattoo based on the user-input including:
∘ determining an electronic circuit design capable of achieving the one or more desired functions, and
∘ designing a final image of the electronic tattoo that mimics the desired appearance and incorporates the electronic circuit design.

2. The computer-implemented method of claim 1, wherein the user-input is provided by a user via a user-interface.

3. The computer-implemented method of any one of the preceding claims, wherein determining the electronic circuit design comprises applying a circuit design algorithm configured to generate circuit parameters for achieving the one or more desired functions.

4. The computer-implemented method of claim 3, wherein generating circuit parameters includes:
- identifying circuit component candidates which fulfill the desired functions,
- identifying electrical connections between circuit component candidates, and
- identifying constraints.

5. The computer-implemented method of claim 4, wherein identifying circuit component candidates includes:
- determining circuit component candidates from circuit components stored in a component database which fulfill the desired functions.

6. The computer-implemented method of any one of claims 3 to 5, wherein designing the final image of the electronic tattoo comprises applying a visual design algorithm configured to:
- obtain the previously generated circuit parameters,
- create a target image which includes determining visual and/or aesthetic features of the desired appearance,
- incorporate the circuit parameters into the target image, and
- create the final image of the electronic tattoo by filling out part of or all remaining sections of the target image with non-conductive tattoo component ink.

7. The computer-implemented method of claim 6, wherein incorporating the circuit parameters into the target image includes:
- selecting final circuit components and placing them into the target image, and
- placing the identified electrical connections into the target image.

8. The computer-implemented method of claim 7, wherein selecting final circuit components includes:
- comparing visual and/or aesthetic features of the circuit component candidates with the visual and/or aesthetic features of the desired appearance,
- selecting those circuit components from the circuit component candidates which closest match the visual and/or aesthetic features of the desired appearance based on the comparison,
- altering sections of the target image to match the visual and/or aesthetic features of those circuit components for which no circuit component candidates are present which match the visual and/or aesthetic features of the desired appearance.

9. The computer-implemented method of claim 8, wherein the visual and/or aesthetic features of the circuit component candidates include one or more of colour, shape and/or size of the circuit component candidates.

10. The computer-implemented method of any one of the preceding claims, further comprising:
- generating user instructions for producing the electronic tattoo based on the final image of the electronic tattoo,
- communicating the user instructions to the user.

11. The computer-implemented method of claim 10, wherein generating user instructions includes applying an instructions generation algorithm configured to:
- generate a text-based list of instructions in natural language, and/or
- generate a sequence of step-by-step images of the electronic tattoo, and/or
- project the final image of the electronic tattoo at a desired location.

12. The computer-implemented method of any one of the preceding claims further comprising:
- obtaining a target location for application of the electronic tattoo,
- collecting physiological data on the target location,
- considering the collected physiological data during determining the electronic circuit design and/or during designing the final image of the electronic tattoo.

13. The computer-implemented method of claim 12, wherein the target location is obtained via user-input on a desired location and/or, wherein the target location is obtained from a look-up table based on the desired function.

14. A storage media comprising instructions that when executed are adapted to cause a computing device to perform the method steps of any one of the preceding claims.

15. A tattoo design system for designing a user-specific electronic tattoo comprising the storage media of claim 14 and a computing device configured to perform the method steps of any one of claims 1-14.
